(19) Europäisches Patentamt
European Patent Office
Office européen des brevets

(11) Numéro de publication : **0 000 452**
**B1**

(12) # FASCICULE DE BREVET EUROPÉEN

(45) Date de publication du fascicule du brevet : **06.05.81**

(21) Numéro de dépôt : **78400026.7**

(22) Date de dépôt : **20.06.78**

(51) Int. Cl.³ : **C 07 D271/06**, C 07 D403/12, C 07 D401/12, C 07 D403/04, A 61 K 31/41// C07C103/75, C07C154/00

(54) **Dérivés d'oxadiazole-1,2,4, leur préparation et leur application en thérapeutique.**

(30) Priorité : **12.07.77 FR 7721447**
**12.07.77 FR 7721447**

(43) Date de publication de la demande : **24.01.79 (Bulletin 79/02)**

(45) Mention de la délivrance du brevet : **06.05.81 Bulletin 81/18**

(84) Etats contractants désignés : **BE CH DE FR GB LU NL SE**

(56) Documents cités :
**DE - A - 2 461 882**

**FR - A - 2 100 914**

**ZEITSCHRIFT FUER CHEMIE 1974, 14 (3) pages 94-95.**
**G. WESTPHAL et R. SCHMIDT « Reaktionen an 3-Amino-1,2,4-oxadiazolen »**

(73) Titulaire : **SYNTHELABO**
**1, avenue de Villars**
**F-75341 Paris Cedex 07 (FR)**

(72) Inventeur : **Dimsdale, Michael John**
**8, Résidence du Moulin de la Planche**
**F-91120 Villebon S/Yvette (FR)**

(74) Mandataire : **Thouret-Lemaitre, Elisabeth**
**Service Brevets - SYNTHELABO 58, rue de la Glacière**
**F-75621 Paris Cedex 13 (FR)**

**EP 0 000 452 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

Dérivés d'oxadiazole-1,2,4, leur préparation et leur application en thérapeutique

La présente invention concerne des dérivés d'oxadiazole, leur préparation et leur application en thérapeutique.

Des dérivés d'oxadiazole ont déjà été décrits dans la littérature, en particulier dans les trois documents suivants :

— l'extrait de « Zeitschrift für Chemie », 1974, 14 (3) pages 94-95 décrit des dérivés d'oxadiazole de formule

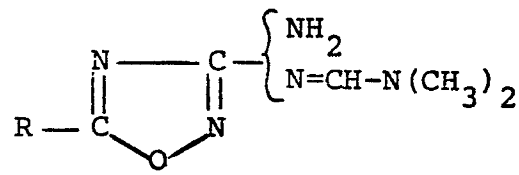

dans laquelle R est $C_6H_5$, $CH_2C_6H_5$ ou $CH(C_6H_5)_2$ ;

— la demande de brevet DE-A-2 461 882 est relative à un procédé de préparation de 3-amino-1,2,4-oxadiazoles dont plusieurs ont été antérieurement décrits dans la littérature comme produits chimiques et comme agents antiinflammatoires ;

— enfin la demande de brevet français n° 2 100 914 concerne des 3-amino-5-benzyl (substitué ou non) -oxadiazoles, utiles comme antiinflammatoires.

La Demanderesse a trouvé que les composés répondant à la formule (I) définie ci-après possèdent une activité antihypertensive.

Les composés de l'invention répondent à la formule (I)

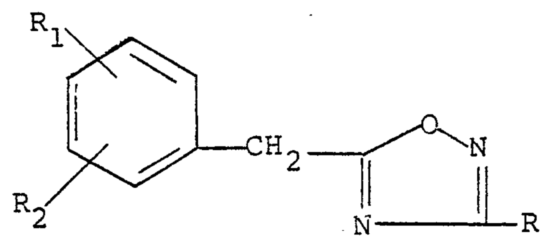

dans laquelle

$R_1$ et $R_2$ sont chacun indépendamment l'un de l'autre un atome d'halogène ou un radical alkyle,

R est

— soit un radical $NR_3R_4$ dans lequel $R_3$ et $R_4$ sont chacun, indépendamment l'un de l'autre, un atome d'hydrogène ou un radical alkyle ;

— soit un radical -N = CH-$NR_5R_6$ dans lequel $R_5$ et $R_6$ sont chacun, indépendamment l'un de l'autre, un radical alkyle, un radical hydroxyalkyle ou $NR_5R_6$ forment un radical

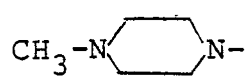

les radicaux alkyles ayant de 1 à 4 atomes de carbone.

Certains composés de l'invention forment des sels d'addition aux acides pharmaceutiquement acceptables ; ces sels font partie de l'invention.

Un groupe particulier de composés est formé par ceux pour lesquels $R_1$ et $R_2$ représentent chacun, indépendamment l'un de l'autre, un atome de chlore, un atome de fluor ou le radical méthyle.

Selon l'invention on prépare les composés en faisant réagir un composé de formule (II)

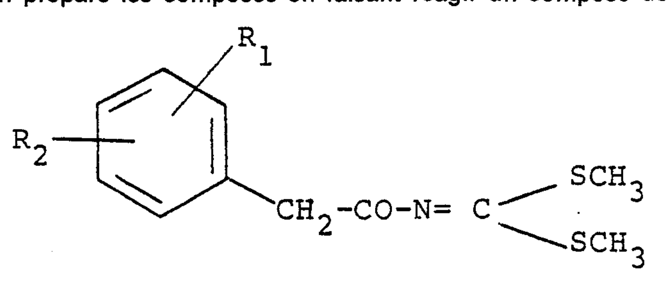

(II)

avec de l'ammoniac ou une amine $HNR_3R_4$ (III), puis l'intermédiaire obtenu avec de l'hydroxylamine.

La réaction est effectuée dans un solvant tel que un alcool inférieur, l'acétone, le benzène, un éther, le chloroforme, mais de préférence dans du méthanol.

On peut aussi préparer les composés de l'invention (I) dans lesquels $R = NH_2$ en faisant réagir un composé de formule (V)

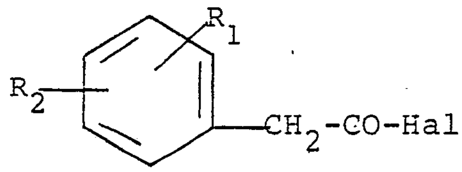

(V)

avec le cyanamide $NH_2CN$, puis l'intermédiaire obtenu avec de l'hydroxylamine.

La première partie de la réaction est effectuée dans un mélange alcalinisé d'eau et d'acétone, à une température allant de 0 à 10 °C.

La seconde partie de la réaction est réalisée dans un solvant polaire, tel qu'un alcool, en présence d'une base tertiaire, sans isolation de l'intermédiaire.

Les composés dans lesquels $R = -N = CH-NR_5R_6$ sont préparés à partir du composé (I) correspondant dans lequel $R = NH_2$ soit par réaction avec un composé

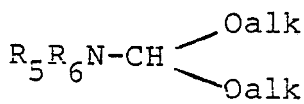

(IV) ou avec le composé $(alkO)_3CH$

puis une amine $R_5R_6NH$.

La réaction avec le composé (IV) est effectuée dans un solvant tel que un éther, le chloroforme, un hydrocarbure tel que le benzène, un alcool inférieur, mais de préférence dans du chloroforme.

Les schémas réactionnels sont les suivants :

Schéma 1

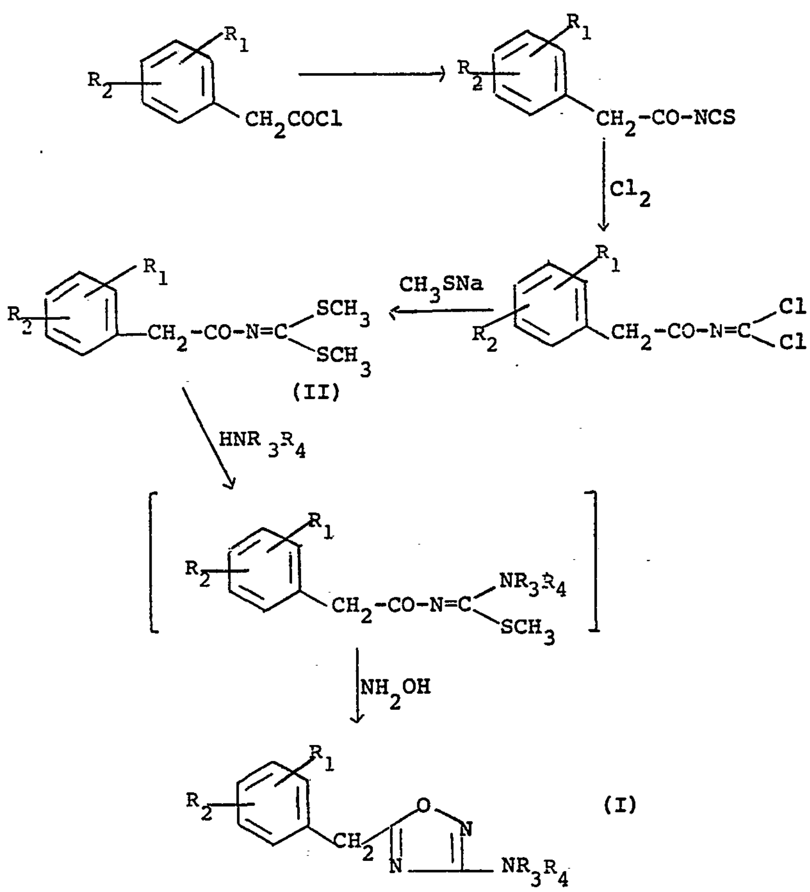


(II)

(I)

Les composés (II) sont nouveaux et font partie de l'invention.

Ils sont obtenus par la méthode de F. Eloy et A. Van Oventraeten [Chimie Thér. 4, 9 (1969)] à partir du chlorure d'acide correspondant.

Schéma 2

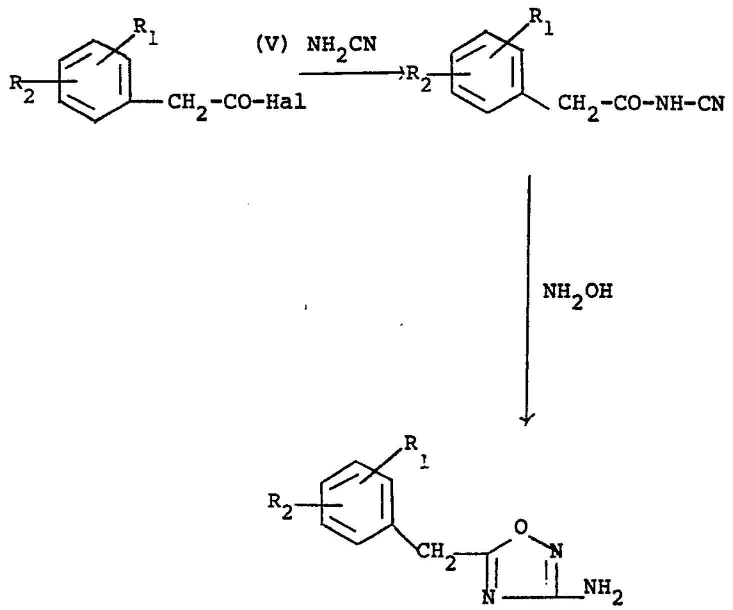


Les composés (V) sont connus et décrits dans la littérature.

Ils sont obtenus à partir du toluène substitué de manière classique (passage par le bromure, le cyanure et l'acide).

Schéma 3

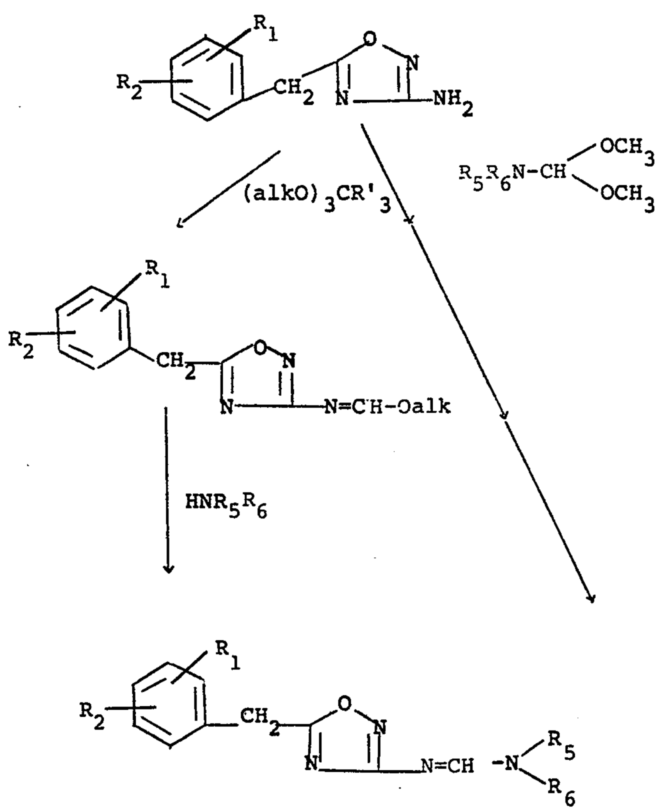

Les exemples suivants illustrent l'invention.

Les analyses et les spectres IR et RMN ont confirmé la structure des composés.

Exemple 1 : Méthylamino-3 (dichloro-2,6-benzyl)-5 oxadiazole-1, 2, 4.

Dans une solution de 6 g (0,019 mole) de N-[bis-diméthylthio]-méthylène (dichloro-2,6 phényl)-acétamide dans 15 $cm^3$ de méthanol on ajoute 12 $cm^3$ de méthylamine en solution dans du méthanol.

On agite 2 heures. On verse cette solution dans une solution de 6,75 g (0,097 mole) de chlorhydrate d'hydroxylamine et de 19 $cm^3$ d'une solution 5,08 molaire de méthanolate de sodium.

On laisse 48 heures à la température ambiante. On concentre à siccité. On reprend le solide formé par $CH_2Cl_2$.

On chromatographie le produit sur une colonne de silice.

F = 128,9 °C.

Exemple 2 : [N,N-bis (hydroxy-2 éthyl)-formamidino]-3 (dichloro-2,6 benzyl)-5 oxadiazole-1, 2, 4 et son chlorhydrate.

1. (Ethoxyméthylène-amino)-3 (dichloro-2,6 benzyl)-5 oxadiazole-1, 2, 4.

On chauffe à l'air libre à 150 °C, pendant 3 heures, 1 g d'amino-3 (dichloro-2,6 benzyl)-5 oxadiazole-1, 2, 4 et quelques ml d'orthoformiate d'éthyle en quantité juste suffisante pour assurer une légère agitation. On chasse sous pression réduite l'excès d'orthoformiate d'éthyle. Le résidu est cristallisé dans de l'éther.

F = 75 °C.

2. [N,N-bis (hydroxy-2 éthyl)-formamidino]-3 (dichloro-2,6 benzyl)-5 oxadiazole-1, 2, 4 et son chlorhydrate.

On dissout 5 g d'(éthoxyméthylène-amino)-3 (dichloro-2,6 benzyl)-5 oxadiazole-1, 2, 4 et 2 g de diéthanolamine dans 70 ml de THF.

Après un repos d'une nuit, le THF est évaporé.

Le résidu est trituré dans de l'éther contenant 10 % de chlorure de méthylène.

Le solide obtenu est cristallisé dans un mélange éther isopropylique/isopropanol 70/30.

F = 108,8 °C.

Le monochlorhydrate fond à 257 °C.

Exemple 3 : Amino-3 (dichloro-2,6 benzyl)-5 oxiadiazole-1,2,4.

On dissout 4,2 g (0,1 mole) de cyanamide dans 96 ml d'eau et 9,6 ml de soude concentrée. On refroidit à 0-5 °C dans un bain de glace et de sel et on ajoute lentement une solution de 23,5 g (0,1 mole) du chlorure de l'acide (dichloro-2,6 phényl)-2 acétique dans 50 ml d'acétone, de façon à ce que la température interne du mélange reste inférieure à 5°. On maintient le pH de la solution entre 10 et 11 à l'aide de quelques gouttes de soude concentrée.

Quand tout le chlorure d'acide a été ajouté, on agite pendant 1 heure le mélange réactionnel en vérifiant que le pH et la température ne se modifient plus.

On acidifie alors avec de l'acide chlorhydrique 6N à 0° ; l'intermédiaire précipite ; on l'essore ou on le dissout dans du chloroforme. Dans ce cas, on lave la solution avec de l'eau, on la sèche sur sulfate de magnésium et on la concentre à siccité.

L'intermédiaire brut est dissous dans 60 ml d'éthanol et on ajoute la solution obtenue à une suspension de 10,75 g (0,15 mole) de chlorhydrate d'hydroxylamine dans 25 ml de pyridine.

La réaction est lente et très légèrement exothermique. On laisse la réaction se faire pendant une nuit. La température se stabilise en général aux environs de 40°. On essore le produit précipité, puis on le lave avec de l'éthanol et de l'éther.

Le filtrat est concentré à siccité, puis repris dans l'eau et alcalinisé avec de la soude 2N. Un deuxième jet d'oxadiazole précipite ; on l'essore et le lave avec de l'éthanol et de l'éther. On recristallise dans de l'éthanol l'amino-3 (dichloro-2,6 benzyl)-5 oxadiazole-1, 2, 4 qui fond à 185 °C.

Dans le tableau I suivant sont représentés les composés de l'invention qui ont été préparés à titre d'exemples selon l'une des méthodes décrites ci-dessus.

HCl = chlorhydrate
$MeSO_3H$ = méthanesulfonate

TABLEAU I

| Composé n° | $R_1$ | $R_2$ | R | F (°C) |
|---|---|---|---|---|
| 1 | Cl-2 | Cl-6 | $N(CH_3)_2$ | 110,3 |
| 2 (ex 1) | Cl-2 | Cl-6 | $NHCH_3$ | 128,9 |
| 3 | Cl-2 | Cl-6 | 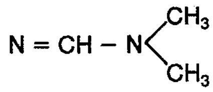 | 138,5-139,5<br>HCl = 208-12 |
| 4 | Cl-2 | Cl-6 | 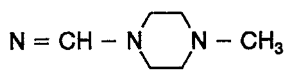 | 113<br>HCl = 257 |
| 5 (ex 2) | Cl-2 | Cl-6 | 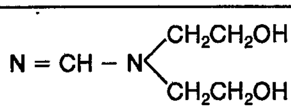 | 108,8 |
| 6 | Cl-2 | Cl-6 | 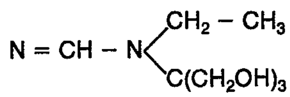 | 144,6 |
| 7 | $CH_3$-2 | $CH_3$-6 | 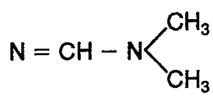 | 89,1 |
| 8 | Cl-2 | F-6 | 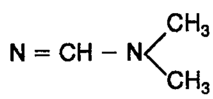 | 91 |
| 9 | F-2 | F-6 | $NH_2$ | 169 |
| 10 | F-2 | F-6 | 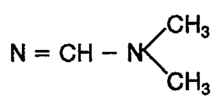 | 75,2 |
| 11 (ex 3) | Cl-2 | Cl-6 | $NH_2$ | 185 |
| 12 | Cl-2 | Cl-5 | $NH_2$ | 153-5 |
| 13 | Cl-3 | Cl-4 | $NH_2$ | 113-113,5 |
| 14 | Cl-2 | Cl-4 | $NH_2$ | 177 |
| 15 | Cl-2 | F-6 | $NH_2$ | 170 |

Les composés de l'invention ont été soumis à des essais pharmacologiques qui ont montré leur activité comme antihypertenseurs.

La toxicité des composés (I) a été déterminée par voie i.p., chez le rat mâle CDI (Charles River) de 100 à 120 g, tenu à jeun 18 heures.

La dose létale 50 % (DL 50) est indiquée dans le tableau II.

L'activité antihypertensive est évaluée chez des rats mâles spontanément hypertendus selon la méthode de Gerold et Tschirky (Arzneim. Forsch. 1968, *18*, 1285). La pression systolique est mesurée par captage du pouls au niveau de l'artère caudale.

A la dose de 10 mg/kg, (5 mg/jour administrés 2 jours de suite par voie orale) on observe les diminutions de la pression sanguine et on les détermine au bout de 4 et 24 heures.

Les résultats obtenus pour certains composés représentatifs de l'invention sont indiqués dans le tableau II.

TABLEAU II

| Composé | DL 50 (mg/kg) i.p. rat | Activité antihypertensive % | | |
|---|---|---|---|---|
| | | dose (mg/kg) p.o. | 4 h | 24 h |
| 1 | – | 20 | – 33 | – 11 |
| 2 | – | 20 | – 18 | – 9 |
| 3 | > 500 | 20 | – 29 | – 15 |
| 4 | < 500 | 20 | – 29 | |
| 5 | ⩾ 500 | 20 | – 32 | |
| 7 | | 20 | – 22 | – 13 |
| 8 | ⩽ 500 | 5 | – 26 | – 6 |
| 9 | | 5 | – 18 | – 1 |
| 11 | 250 | 5 | – 18 | – 3 |
| 12 | ⩾ 500 | 5 | – 18 | – 3 |

On doit noter que, dans l'épreuve d'actimétrie chez la souris, l'action sédative des composés de l'invention s'est révélée négligeable.

Les résultats des essais pharmacologiques montrent que les composés de l'invention sont utilisables comme médicaments, en thérapeutique humaine et vétérinaire, dans le domaine cardiovasculaire, comme antihypertenseurs.

Ils sont particulièrement utilisés dans le traitement de toutes les formes d'hypertension essentielle ou secondaire.

L'invention comprend, par conséquent, toutes compositions pharmaceutiques renfermant au moins l'un des composés (I) comme principe actif, en association avec tous excipients appropriés à leur administration, principalement par voie orale, mais aussi par voie endorectale ou parentérale.

La posologie quotidienne par voie orale peut varier de 4 à 100 mg.

**Revendications**

1. Dérivés d'oxadiazole, caractérisés en ce qu'ils répondent à la formule

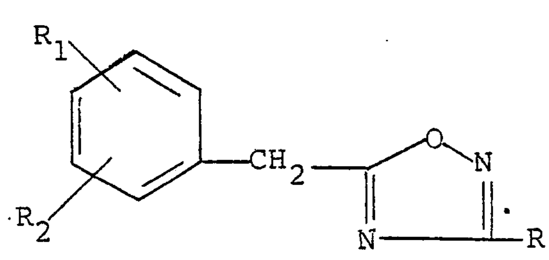

dans laquelle

$R_1$ et $R_2$ sont chacun indépendamment l'un de l'autre un atome d'halogène ou un radical alkyle,

R est

— soit un radical $NR_3R_4$ dans lequel $R_3$ et $R_4$ sont chacun, indépendamment l'un de l'autre, un atome d'hydrogène ou un radical alkyle ;

— soit un radical $-N = CH-NR_5R_6$ dans lequel $R_5$ et $R_6$ sont chacun, indépendamment l'un de l'autre, un radical alkyle, un radical hydroxyalkyle ou $NR_5R_6$ forment un radical

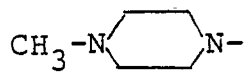

les radicaux alkyles ayant de 1 à 4 atomes de carbone.

2. Sels d'addition que forment certains composés (I) avec les acides pharmaceutiquement acceptables.

3. Dérivés selon la revendication 1, dans lesquels $R_1$ et $R_2$ représentent indépendamment l'un de l'autre un atome de chlore ou de fluor ou le radical méthyle.

4. Dérivés selon la revendication 1, caractérisés par le fait que les radicaux alkyles des groupes R sont des radicaux méthyles.

5. Procédé de préparation des composés selon la revendication 1, procédé caractérisé en ce qu'on fait réagir un composé de formule

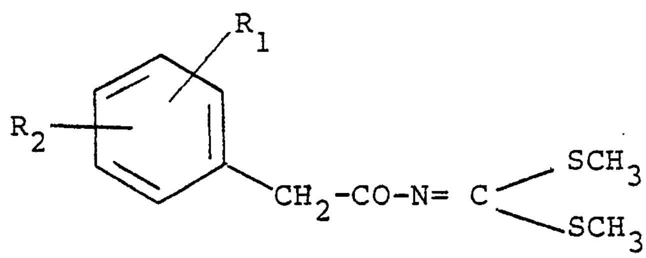

(II)

avec de l'ammoniac ou une amine $HRN_3R_4$ (III), puis on fait réagir l'intermédiaire obtenu avec de l'hydroxylamine,

et, pour obtenir les composés (I) dans lesquels R est $-N = CH-NR_5R_6$ on fait réagir le composé (I) dans lequel R est $NH_2$ soit avec un composé $(alkO)_3CH$ puis l'intermédiaire obtenu avec une amine $HNR_5R_6$ soit avec un composé

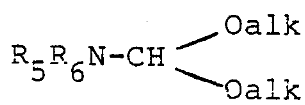

les radicaux ayant les significations données dans la revendication 1.

6. Procédé de préparation des composés (I) dans lesquels R est $NH_2$, procédé caractérisé en ce qu'on fait réagir un composé (V)

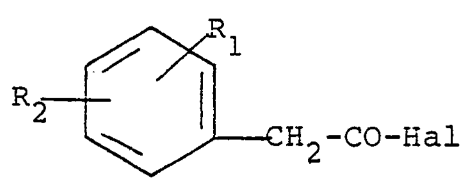

(V)

avec le cyanamide puis l'intermédiaire obtenu avec de l'hydroxylamine.

7. Composition pharmaceutique caractérisée en ce qu'elle contient un composé tel que spécifié dans l'une quelconque des revendications 1 à 4.

## Claims

1. Oxadiazole derivatives of the formula (I)

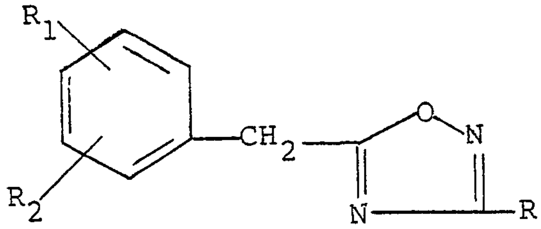

(I)

in which
each of $R_1$ and $R_2$ independently represents a halogen atom or an alkyl radical,
R represents
either a $NR_3R_4$ radical in which each of $R_3$ and $R_4$ independently represents a hydrogen atom or an alkyl radical,
or a $-N=CH-NR_5R_6$ radical in which each of $R_5$ and $R_6$ independently represents an alkyl radical, a hydroxyalkyl radical or N, $R_5$ and $R_6$ together form a

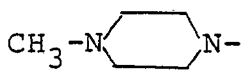

radical, each of the above-specified alkyl radicals having 1 to 4 carbon atoms.

2. Pharmaceutically acceptable acid addition salts of certain compounds of formula (I).

3. Derivatives according to claim 1, in which each of $R_1$ and $R_2$ independently represents a chlorine atom, a fluorine atom or a methyl radical.

4. Derivatives according to claim 1, wherein the alkyl radicals represented by R are methyl radicals.

5. A process for the preparation of compounds according to claim 1, which process comprises reacting a compound of the formula

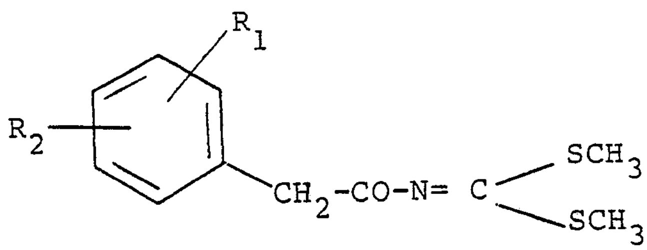

(II)

with ammonia or an amine $HNR_3R_4$ (III) to produce an intermediate and reacting said intermediate with hydroxylamine, and for obtaining compounds of the formula (I) in which R represents $-N=CH-NR_5R_6$, reacting the compound of formula (I), in which R represents $NH_2$,

— either with a compound of formula $(alkO)_3CH$ to produce an intermediate and reacting said intermediate with an amide of formula $HNR_5R_6$,

— or with a compound of formula

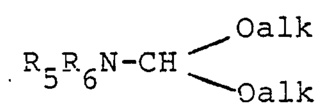

the radicals having the meanings given in claim 1.

6. A process for the preparation of compounds of formula (I) in which R is $NH_2$, which process comprises reacting a compound of formula (V)

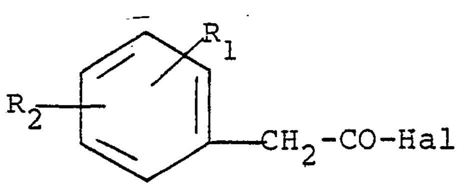

(V)

with cyanamide to produce an intermediate and reacting said intermediate with hydroxylamine.

7. A pharmaceutical composition comprising a compound according to anyone of claims 1 to 4.

**Ansprüche**

1. Oxadiazolderivate der allgemeinen Formel (I)

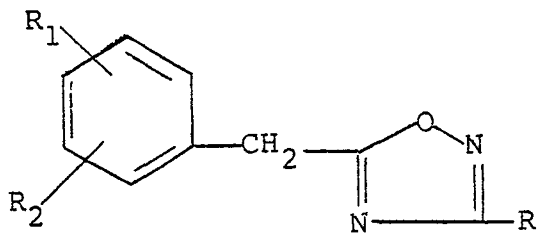

(I)

in der
$R_1$ und $R_2$ unabhängig voneinander Halogen-atome oder Alkylgruppen bedeuten, und
R entweder eine Gruppe der Formel $NR_3R_4$, in der
$R_3$ und $R_4$ unabhängig voneinander für Wasserstoffatome oder Alkylgruppen stehen,
oder eine Gruppe der Formel $-N = CH-NR_5R_6$, in der $R_5$ und $R_6$ unabhängig voneinander für Alkyl-oder Hydroxyalkylgruppen stehen, oder N, $R_5$ und $R_6$ gemeinsam für eine Gruppe

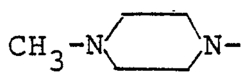

stehen, bedeutet

wobei die Alkylgruppen 1 bis 4 Kohlenstoffatome aufweisen.

2. Additionssalze, die bestimmte Verbindungen der allgemeinen Formel (I) mit pharmazeutisch annehmbaren Säuren bilden.

3. Derivate nach Anspruch 1, dadurch gekennzeichnet, daß $R_1$ und $R_2$ unabhängig voneinander Chloratome, Fluoratome oder Methylgruppen bedeuten.

4. Derivate nach Anspruch 1, dadurch gekennzeichnet, daß die Alkylgruppen der Gruppen R Methylgruppen sind.

5. Verfahren zur Herstellung der Verbindungen nach Anspruch 1, dadurch gekennzeichnet, daß man eine Verbindung der allgemeinen Formel (II)

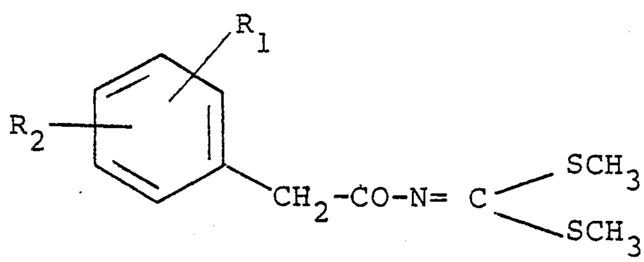

(II)

mit Ammoniak oder einem Amin der allgemeinen Formel $HNR_3R_4$ (III) umsetzt, dann das erhaltene Zwischenprodukt mit Hydroxylamin umsetzt und
zur Bildung der Verbindungen der allgemeinen Formel I, in der R eine Gruppe der Formel $-N = CH-NR_5R_6$ darstellt, eine Verbindung der Formel I, in der R für eine $NH_2$-Gruppe steht,
entweder mit einer Verbindung der allgemeinen Formel $(alkO)_3CH$ und dann das erhaltene Zwischenprodukt mit einem Amin der allgemeinen Formel $HNR_5R_6$ umsetzt
oder mit einer Verbindung der allgemeinen Formel

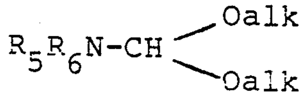

umsetzt, wobei die Substituenten die in Anspruch 1 angegebenen Bedeutungen besitzen.

6. Verfahren zur Herstellung der Verbindungen der allgemeinen Formel (I), in der R für eine $NH_2$-gruppe steht, dadurch gekennzeichnet, daß man eine Verbindung der Formel (V)

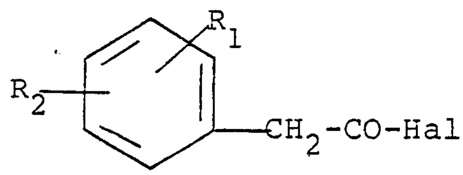

(V)

mit Cyanamid umsetzt und dann das erhaltene Zwischenprodukt mit Hydroxylamin umsetzt.

7. Pharmazeutische Zubereitung, dadurch gekennzeichnet, daß sie eine Verbindung gemäß den Ansprüchen 1 bis 6 enthält.